# EUROPEAN PATENT APPLICATION

(11) **EP 4 151 159 A1**
(43) Date of publication of application: **22.03.2023**
(21) Application number: 21315162.4
(22) Date of filing: 16.09.2021
(51) Int. Cl.: A61B 17/00, A61M 25/00, A61M 25/01

(54) **APPARATUS FOR PERFORMING A SURGICAL PROCEDURE**

(71) Applicant: Caranx Medical SAS, 75003 Paris (FR)
(72) Inventor: SEJOR, Eric, 06000 Nice (FR); SMITS, Jonas Victor Harmen, 3360 Bierbeek (BE); BERTHET-RAYNE, Pierre, 06800 Cagnes-sur-Mer (FR)
(74) Representative: Hepp Wenger Ryffel AG

(57) **Abstract**

The invention relates to an apparatus for insertion into a patient's body in a minimally invasive procedure. The apparatus comprises an elongated support and a trigger element. The elongated support comprises a first section and a second section which are movable with respect to each other between a first position and a second position. The elongated support has a locking arrangement which is configured to (i) reversibly increase friction between the first section and the second section in a locked configuration, thereby inhibiting a relative movement between the first section and second section due to the increased friction and locking the elongated support in at least one of the first position and second position. The locking arrangement is also configured to (ii) decrease friction between the first section and second section in a flexible configuration, thereby allowing a relative movement between the first section and second section. The locking arrangement is activatable by the trigger element.

## Description

The invention relates to an apparatus for performing a surgical procedure within the body of a patient, preferably in a body duct of a patient.

For instance, percutaneous minimally invasive surgical procedures are often performed by using an apparatus for carrying or introducing surgical instruments, e.g. guidewires and/or catheters, which are guided through narrow vessels to a target site.

Often the apparatus must traverse long distances within a patient's body ducts along twists and windings. In order to follow the turns of the body duct, the device must be bendable in a radial direction to mimic the curvatures of the body ducts.

US 6 3027 905 discloses a highly articulated robotic probe for minimally invasive surgery. The probe comprises an outer sleeve with a plurality of links and is provided with manoeuvrability by conventional actuation of a plurality of wires.

In the prior art, guidewires are often inserted into an apparatus such that a flexible tip of the apparatus can assume a curvature to mimic the bending of the blood vessels. However, this process requires a plurality of bending steps and hence is very time-consuming and, moreover, sometimes the guidewire shape cannot be shaped to conform to specific shapes of body ducts.

Another hurdle in such surgical interventions is that in certain situations the formation of a radial curvature of such an apparatus is not desirable, as deflection or slippage of the device could, for example, dislodge a blood clot (thrombus) during a endovascular procedure or cause traction on the feeder vessel of the explored canal or dilacerate the canal during an endoscopy. In other cases, for example, an embolism must be punctured to remove a vascular occlusion which cannot be carried out with a flexible apparatus.

However, increased time required for an operation and/or imprecise surgery pose a significant health risk to patients and should be optimised.

It is an object of the present invention to overcome the drawbacks of the prior art.

This object is solved by an apparatus for insertion into the body of a patient according to the claims.

The apparatus for insertion into a patient's body, preferably a body duct, comprises an elongated support and a trigger element. The apparatus is used useable particularly for minimally invasive procedures, such as a percutaneous or an endocanal procedure. Endoscopical uses can be colonoscopy, bronchoscopy, urethroscopy, cystoscopy, ureteroscopy. In percutaneous procedures, the apparatus may be used as a catheter. The elongated support comprises at least one first section and at least one second section which are movable with respect to each other between a first position and a second position. The elongated support has at least one locking arrangement which is configured to reversibly:
(i) increase friction between the first section and second sectioh in a locked configuration, thereby inhibiting a relative movement between the first and second section due to increased friction and locking the elongated support in at least one of the first position and second position, and
(ii) decrease friction between the first section and the second section in a flexible configuration, thereby allowing a relative movement between the first section and the second section.

The locking arrangement is activatable by the trigger element.

In this context, friction between the first and second section refers to a direct but also an indirect contact between the first and second section, i.e. an intermediate layer also may be arranged between the first and second section.

The first and second sections may be facing each other in the radial direction of the apparatus.

The elongated support can be a guidewire, a catheter, a growing and/or evertable structure or a surgical device configured to carry out surgical procedures within a patient's body.

The elongated support can comprise a bendable section which is bendable, preferably in a continuous range of intermediate positions. By bending some or all of the first and second sections may be moved between the first and second positions(s). In the first position, the bendable section may be at least partially straight. In the second position the bendable section may be at least partially curved and can define a curvature with a maximum uniform bending angle.

In a preferred embodiment of the apparatus the elongated support can be reversibly locked in a plurality of intermediate positions between the first position and second position. The inhibition and/or activation of relative movement by increased friction and/or decreased friction can preferably be set from a continuous range of frictional states.

The first section and second section of the apparatus' can be formed on or by separate elements in a longitudinal direction of the apparatus which may also be movably connected to each other. In such an embodiment the relative movement may allow for a deflection of the elongated support while the first section and second section are still mechanically connected.

When locking the first and second position by an increase in friction, the locking arrangement prevents or inhibits a bending or a straightening of certain or all parts of the elongated support, in particular at forces occurring when advancing the apparatus within a patient's body, in particular preferably due to advancement in a patient's body ducts or when an unlocked distal part shall be bent while more locked proximal parts shall remain in a straight or curved configuration. Locking in the first and second position can also prevent or inhibit bending or straightening of certain or all parts of the elongated support when sliding a different device around or within the elongated support. This can be e.g. a device arranged coaxially to the elongated support arranged device such as a replacement valve.

In a different embodiment the first section and second section of the apparatus are defined by elements which are not connected to each other and which are translationally movable relative to each other.

The trigger element can be a supply of an electric voltage such that the locking arrangement can be activated by supplying a voltage difference to the first and second section to cause an increased electrostatic adherence between the first and second section, analogous to an electromagnetic clutch or brake.

Such a brake is e.g. disclosed by Hinchet et al., DextrES: Wearable Haptic Feedback for Grasping in VR via a Thin Form-Factor Electrostatic Brake, UIST '18: Proceedings of the 31st Annual ACM Symposium on User Interface Software and Technology October 2018 Pages 901-912.

The locking arrangement can be activatable by positioning the trigger element to an appropriate position relative to the elongated support, in particular by relative movement in a longitudinal direction of the elongated support. In the appropriate position the trigger element may form an electric contact to the first or to the second section.

The trigger element can, however, also be expandable and/or collapsible for pushing the first and second section together and thereby increase friction, in particular in a radial direction of the apparatus, if being activated and/or deactivated.

The apparatus can be torsionally rigid such that the rotation of a section of the apparatus at an angle x° at a certain longitudinal location results in essentially the same rotation of an angle x° at other sections of the apparatus along the longitudinal axis.

The trigger element can be an elongated element and one of the trigger element or the elongated support can be nested within the other of the trigger element and the elongated support. The trigger element and the elongated support can be slidable relative with respect to each other, e.g. in a longitudinal or helical direction of the apparatus. The apparatus can comprise linear and/or rotational actuators configured to carry out relative movement of the trigger element and/or elongated support with respect to each other.

The bendable section can encompass the first section(s) and the second section(s) and be bendable along a preferably uniform bending angle.

The apparatus can comprise multiple bendable sections with multiple first and second sections. Multiple bendable sections can be arranged in the longitudinal direction of the apparatus. The bendable sections can be arranged to partially overlap along the longitudinal direction of the apparatus.

The apparatus can comprise multiple locking arrangements with multiple first sections and second sections. The multiple locking arrangements can be arranged to partially overlap along the longitudinal direction of the apparatus.

The trigger element can be configured to at least partially activate the locking arrangement if advanced in a longitudinal direction of the elongated support to an area neighbouring the bendable section. The trigger element can be configured such that displacing, in particular retracting, the trigger element in the longitudinal direction away from the bendable section of the elongated support at least partially deactivates the locking arrangement.

The term "partially activate" and "partially deactivate" in this context means that only a local subsection of the locking arrangement is activated or deactivated. In particular this can be the subsection along which the trigger element is movable/positionable.

The partial activation/deactivation of bendable sections can occur only in a specific local range or on in multiple ranges. The specific range of activation/deactivation of the locking arrangement can be adjusted by the distance along which the trigger element is advanced and/or retracted in the longitudinal direction of the apparatus.

The apparatus can comprise at least one navigation element, in particular two pull-wires, in particular preferably three pull-wires. The navigation element can preferably apply bending forces in radial directions of the apparatus for deflecting and/or steering the apparatus, when the first section and the second section are in the flexible configuration.

The pull-wires are preferably attached at the distal end region of the apparatus. The pull-wires are preferably placed in, preferably different, radially outer areas of the device such that a directed bending force can be applied by pulling the pull-wires.

Alternatively a bending force in an opposite direction to pulling might be applied by advancing the pull-wires having sufficient column strength.

Alternatively the navigation element can comprise or consist of of a piezo actuation element, a magnetic actuation, in particular external magnetic actuation, a micromechanical alloy, or a shape memory alloy, in particular Nitinol. The navigation element can be expandable and/or collapsible, in particular in the longitudinal direction of the apparatus.

A piezo actuation element can be configured to be expandable or compressible in the longitudinal direction of the apparatus, in particular on one side of the apparatus. An asymmetric change in longitudinal expansion of the apparatus due to the piezo actuation element can deflect the apparatus to assume a curvature.

A bent configuration of the apparatus with a complex geometric form can comprise at least two different curvatures or straight parts in the bendable section. The sections can extend from 0.01 cm to 15 cm. These bendable sections preferably can be locked individually.

The trigger element can be used to lock the bendable section in an iterative process. In a first step a part of the bendable section which is in the flexible configuration can be deflected by advancing it against a body section or by deflecting it through the navigation element. Afterwards, a desired part of the deflected bendable section can be converted to the locked configuration. The part of the bendable section which is. now in the locked configuration cannot be deflected any longer by operating the navigation elements, or in particular by introduction into body ducts. Then, a remaining flexible part of the bendable section can be deflected differently and converted to the locked configuration such that complex geometric forms can be formed by this iterative process.

In a similar way the trigger element can lock also more than one bendable sections in an iterative process.

The iterative process of converting multiple bendable sections to the locked configuration can be carried out by bending a first bendable section to the desired curvature. Then the first bendable section can be converted from the flexible configuration to the locked configuration. The first bendable section can in particular be converted by advancing the trigger element from a position proximal to the first bendable section to a position close to the first bendable section. The first bendable section in the locked configuration is no longer deflectable. A second bendable section distal to the first bendable section can then be bent to a desired curvature, preferably by the navigation element, without deflecting the first bendable section. The second bendable section can then be converted from the flexible configuration to the locked configuration. This procedure can be repeated for multiple, bendable sections to form complex geometric forms.

The apparatus can exert a spring force that returns the bendable section(s) to the straight state if the first and second sections are in the flexible configuration without operating the navigation elements. The resetting spring force can enable easier adjustment of the bend of the bendable section by means of a haptic resistance to the operating movements of a user.

As disclosed above, the locking arrangement can be an electrostatic arrangement. For this purpose, the locking arrangement can have a first conducting layer and a second conducting layer separated by a dielectric layer. The friction between the first conducting layer, the dielectric layer and the second conducting layer is adjustable by applying a difference of electric potential between the first conducting layer and the second conducting layer. In this case the first section and the second section will be formed by the first and the second conducting layer.

The apparatus can comprise multiple conducting layers associated to multiple bendable sections. The conducting layers of different bendable sections can be connected to a voltage source by an individual conducting connector.

One conducting layer may be part of a locking arrangement for multiple bendable sections or separate conducting layers may be associated with several bendable sections.

As explained above, the trigger element can apply the electric potential difference between the first conducting layer and the second conducting layer. Thereby, the locking arrangement can be activated. Removing the electric potential difference applied by the trigger element can deactivate the locking arrangement.

The conducting layers can be arranged along the circumference of the apparatus and in particular concentrically. A plurality of layers may be arranged along the longitudinal direction of the apparatus. The first conducting layer and the second conducting layer can extend essentially over a same surface.

The conducting layers can also form a conductive structure or consist of multiple conductive elements. In particular the conducting layers can comprise a mesh of multiple conductive elements. The conductive elements of one layer can be movably arranged relative to each other such that the flexibility of the apparatus is increased.

A macroscopic geometric form or a surface roughness of the first and/or second section can additionally be provided to increase or decrease friction by additionally providing a form fit.

If a trigger element supplying an electrical voltage is used, it is possible to provide for switching the supply on and off. If the supply is off, the trigger element might be advanced and/or retracted to an area neighbouring the bendable section without activating and/or deactivating the locking arrangement. The locking arrangement can be at least partially activated in such a case if the voltage is switched on. In particular, areas of the bendable section adjacent to the locking element can be locked in the present curvature by supplying, a voltage.

The voltage difference can be applied by an external control element.

The trigger element can comprise one of the conducting layers. In particular a first or second conducting layer can be placed on the trigger element. The trigger element with one conducting layer can be movable relative to the other conducting layer. The first and second conducting layer can also both be placed adjacent to the trigger element and the trigger element can be movable relative to both conducting layers.

The locking arrangement can comprise multiple first conducting layers and/or second conducting layers, and/or preferably dielectric layers, configured so that at least two frictional contacting surfaces are formed.

The dielectric layer can be coated on one of the elements forming the conducting layers. A dielectric layer preferably can be coated to both of the conducting layers so such that the dielectric layer frictionally contact each other. It is also possible to provide a separate dielectric layer between the first and second conductor. Optionally the conducting layer may be covered by a protective layer on the side opposite the dielectric layer.

The dielectric layer can comprise or consist of plastic material like polyimide, polyethylene terephthalate, polyethylene naphthalate, polyphenylene sulphide, polypropylene, polytetrafluoroethylene, polystyrene, and/or polycarbonate.

In some embodiments the dielectric layer or another intermediate layer between the first and second section can be configured to increase the friction in the locked configuration and/or decrease friction in the flexible configuration, e.g. by choice of material or surface structure.

The conducting layers can consist of or comprise conductive metals such as steel, copper, nickel, aluminium, copper, silver, gold, brass, steel, lead, tin, titanium, electrically conductive acrylic or carbon, or shape memory alloys, in particular Nitinol.

It is readily understood that any other material with suitable high and low electric conductivities can be chosen for the conducting layers and insulating layers. Materials suitable for use in electrical devices such as known materials for wires, electrical or electronic components, electromechanical actuators, coils, or capacitors can also be used in the conducting layer and insulating layer.

The apparatus can comprise multiple conducting paths contacting the conducting layers. The apparatus can e.g. comprise a flexible printed circuit board forming conductive paths leading to conducting layers such that individual conducting layers of the apparatus can be selectively supplied with a voltage to activate/deactivate the locking arrangement or locking arrangements, preferably independently of each other.

The apparatus can comprise at least one elongated lumen which preferably extends from a proximal end region of the apparatus to a distal end region of the elongated support. The trigger element can be preferably axially movable within the lumen.

The lumen can comprise a working channel for introducing surgical instruments and/or implants.

The elongated support around the lumen can be configured to be radially stiff so that the lumen does not collapse, irrespective of the curvature of the apparatus.

The apparatus can have an essentially constant profile along its length. Depending on the specific intended use, the profile can have an outer diameter of 3 FR to 90 FR, wherein 3 FR are 1 mm.

The locking arrangement can be positioned along the entire length of the apparatus or only along a partial length of the apparatus.

The maximum bending angle may be 14°, in particular 35°, in particular preferably 45°, along a longitudinal length of the apparatus of 1 cm.

The maximum bending angle is preferably large enough such that the apparatus can be introduced into branched body ducts of a patient at wide angles.

Since the present invention allows a continuous structure (as opposed to an articulated structure) a smooth and continuous change of shape is possible. Bending radii typically in the range between 5 mm to 50 mm are conceivable. If the device is used as a catheter or a guidewire, radii in the order of 10mm-20mm are preferred.

In a preferred embodiment, the locking arrangement can comprise scale elements, in particular at least one group of two radially adjacent scale elements. The first section can comprise a first scale element and/or the second section can comprise a second scale element.

The scale elements can be at least partially overlapping along the longitudinal direction and/or the circumferential direction of the apparatus.

In one embodiment the elongated support may be formed by a structurally independent base layer carrying the scale elements. The scale elements can be attached individually or in groups to the base layer by means of connecting elements.

In another embodiment the elongated support can be constituted by the scale elements. The scale elements can be connected to an adjacent scale element, so that a freely movable joint is created at a connection area.

The elongated support can be enclosed by more than one layer of scale elements.

The scale elements can be configured to be at least partially tiltable away from the longitudinal axis of the apparatus and optionally rotatable in circumferential direction of the apparatus.

The maximum bending angle results from the maximum relative movability of the scale elements in two degrees of freedom so that at the maximum bending angle, a further movement between adjacent scale elements is prevented and further deflection of the apparatus is prevented.

The range of movement can be limited by additional structures, in particular by protrusions or recesses abutting each other when a maximum deflection is reached.

The first section and the second section can be separate from the scale elements.

The first and second section may also be part of the scale elements which can contact each other in the first position and in the second position.

Neighbouring scale elements can define the first and second section which can be spaced apart by a dielectric coating. The friction between the scale elements can be increased to convert an area of the elongated support to a locked configuration by applying a voltage difference to individual scale elements.

The scale elements may consist of or comprise a conductive material. The first and/or second conductive layer(s) as described herein above can be formed by a plurality of scale elements or by a single scale element.

At least some scale elements may be conductively connected in the longitudinal direction and/or the circumferential direction of the apparatus by contacting each other.

The scale elements can be coated with an insulating material or be at least partially surrounded by an insulating layer.

The insulating layer can comprise or be made of a piezoelectric material or other materials with a low electrical conductivity such as insulating plastic, in particular polyimide.

The apparatus may comprise sections wherein essentially all scale elements are conductively connected to each other and to a certain electric potential and other sections where only some or no scale elements are conductively connected to a certain potential.

In such an embodiment of the scale elements, rows of scale elements, or areas of scale elements could be subject to different voltages to form a plurality of frictional surfaces.

The elongated support can have at least one of the following features: (i) the elongated support has a closed surface along the longitudinal direction, (ii) the elongated support is radially stiff, (iii) the elongated support is configured to have a uniform flexural rigidity, and (iv) the elongated support has essentially the same cross-section along its longitudinal axis.

In the flexible configuration the first section and second section may also be configured to be radially flexible. The profile of the apparatus can then be compressed to assume a dented state.

The trigger element can alternatively be configured to actuate a volume modification element of the apparatus. The volume modification element can be expandable and/or collapsible in a radial direction of the apparatus e.g. by inflation pressure or by a piezoelectric element pressing against the first or second section, thereby increasing friction.

The apparatus can comprise an external control element which is configured to be situated outside of the patient in the proximal end region of the apparatus. The control element can be configured to (i) operate the navigation element, (ii) activate or deactivate the trigger element, (iii) optionally to advance and/or retract the elongated support or trigger element relative to the elongated support or trigger element.

According to another aspect of the invention a method for performing a percutaneous or endoluminal procedure is provided, preferably by using an apparatus as described above. The method comprises the following steps:
- Inserting at least a distal end of an apparatus comprising an elongated support and a trigger element into a lumen of a patient.
- Moving a first section of the elongated support with respect to a second section of the elongated support into a desired position, preferably moving between a first position and a second position.
- Reversibly increasing friction between the first section and the second section by activating a locking arrangement of the elongated support with a trigger element. Thereby, at least one pair of the first section and the second section is converted to a locked configuration such that the first and second section are inhibited or prevented from a relative movement between the first section and second section due to an increased friction. Thereby, the elongated support is locked in at least one of the first position and second position.
- In a further step, optionally, the friction between the first section and second section can be reversibly decreased such that the device assumes a flexible configuration and the first section is movable relative to the second section.

The above described method can comprise the step of sliding the trigger element in the form of an elongated element. One of the trigger element and the elongated support can be nested within each other. The trigger element and the elongated support can then be slid relative to each other.

The method can comprise the step of bending a bendable section encompassing the first section and the second section along a continuous, preferably an uniform bending angle. The first position defines a straight arrangement of the bendable section and the second position defines a bent arrangement of the bendable section.

The method can comprise the step of carrying out an iterative process, such that in a first step, the bendable section in the flexible configuration is deflected with the aid of a navigation element to a second position. In a second step, a part of the deflected bendable section is converted to the locked configuration. The part of the bendable section which is now in the locked configuration cannot be deflected any longer by operating the navigation elements. The first step and second step can be carried out iteratively on further parts of the bendable section to sequentially deflect the remaining bendable section (which is still in the flexible configuration) differently and to then convert those parts to the locked configuration. At the end of the iterative procedure, the bendable section may have differently bent parts in the locked configuration.

The method can comprise the step of applying a difference of electric potential between a first conducting layer and a second conducting layer of the locking arrangement, such that the friction between the first conducting layer and the second conducting layer is increased.

The invention will now be described with reference to specific . embodiments and the accompanying drawings, which show:
- Figures 1A and 1B:: an embodiment of an apparatus according to the invention,
- Figure 2A:: a longitudinal cross-section of an embodiment of the apparatus comprising scale elements,
- Figure 2B:: a side view of the embodiment of Fig. 2A,
- Figure 2C:: a radial cross-section of the apparatus according to the embodiment of Fig. 2A and Fig. 2B,
- Figure 3A:: a longitudinal cross-section of an embodiment of the apparatus with a slidable trigger element in a retracted state,
- Figure 3B:: a longitudinal cross-section of the apparatus according to Fig. 3A with the trigger element in an advanced state,
- Figure 3C:: shows an enlarged view of the apparatus according to Fig 3A and Fig. 3B with an evertable trigger element,
- Figures 4A and 4B:: a schematic illustration of degrees of freedom of the apparatus,
- Figures 5A to 5B:: a schematic illustration of conversion of the apparatus from a flexible configuration to a locked curved configuration through an iterative process,
- Figure 5C:: a schematic illustration showing converting parts of a bendable section from the flexible configuration to the locked configuration,
- Figures 6A and 6B:: a specific embodiment of scale elements of the apparatus movable in two degrees of freedom,
- Figure 7A:: a schematic longitudinal cross-section of an embodiment of the apparatus with scale elements,
- Figure 7B:: an alternative embodiment with scale elements
- Figures 8A to 8D:: the schematic mechanism of increasing friction by applying an electrical field,
- Figure 9A to 9B:: an embodiment of the apparatus of Fig. 7B in a first and second position,
- Figure 10A to 10B:: an alternative embodiment of the apparatus with scale elements in two perspective views,
- Figures 11A to 11C:: embodiments of the apparatus with different types of scale elements,
- Figure 12A:: a perspective side view of an alternative embodiment of an apparatus with four conducting layers arranged in circumferential direction,
- Figure 12B:: the apparatus according to Fig. 12A in a cross-sectional view,
- Figure 13A:: an alternative embodiment of an apparatus with conductive layers,
- Figure 13B:: the apparatus according to Fig. 13A in a radial cross-sectional view.

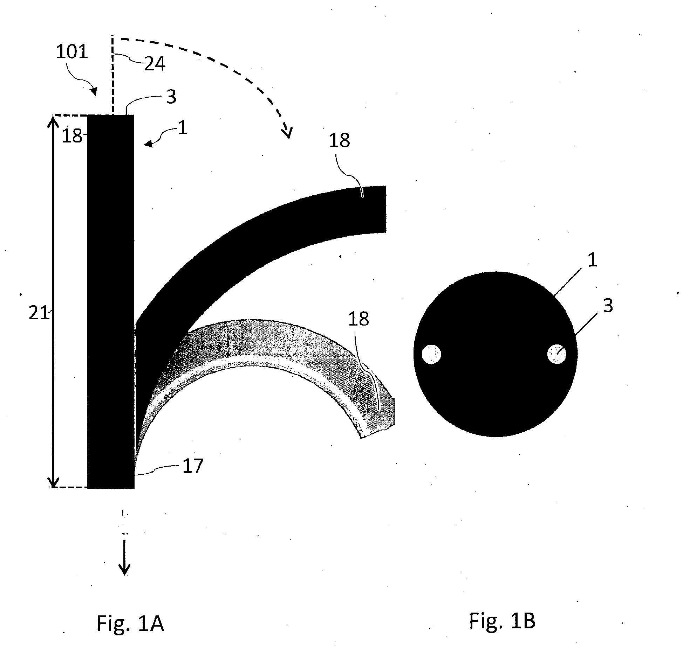

Figure 1A schematically shows an apparatus 101 that is bendable from a longitudinal axis 24 of the apparatus 101. A proximal end region 17 remains close to the axis 24 while a distal end region 18 moves relative away from the axis 24. This is facilitated by a bendable section 21 which bends along a bending angle away from the axis 24. The bending angle is uniform along the bendable section 21 and can be adjusted by pulling or releasing navigation elements 3 which are realised by tendons extending in longitudinal direction of the apparatus 101 (see Fig. 1B).

In this embodiment an elongated support 1 is configured to be flexible such that the deflection of the apparatus 101 in a radial direction can be carried out elastically without plastic deformations. In this embodiment a cross-sectional profile of the apparatus 101 is essentially circular. However, an elliptical or polygonal cross-sectional profile could also be conceived.

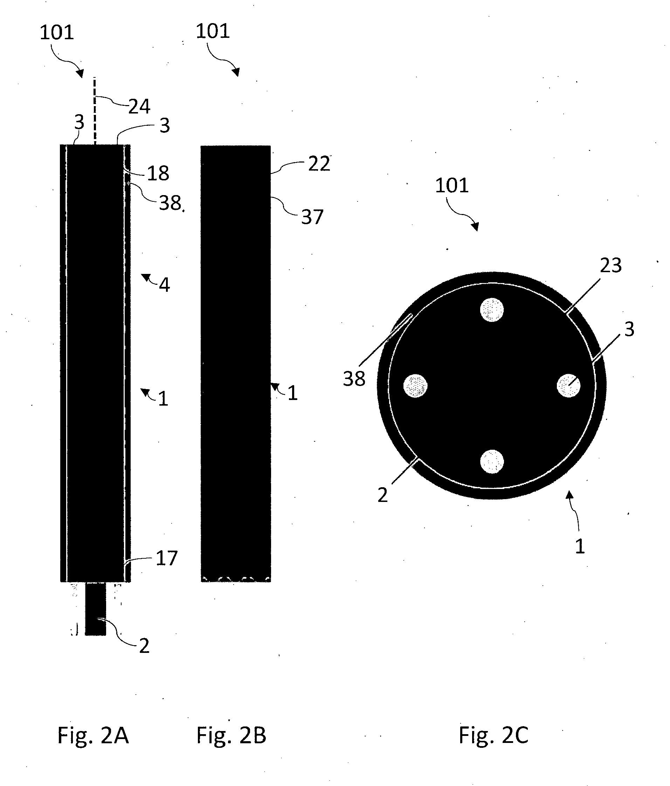

Figure 2A shows a longitudinal cross-section of a specific embodiment of an apparatus 101.

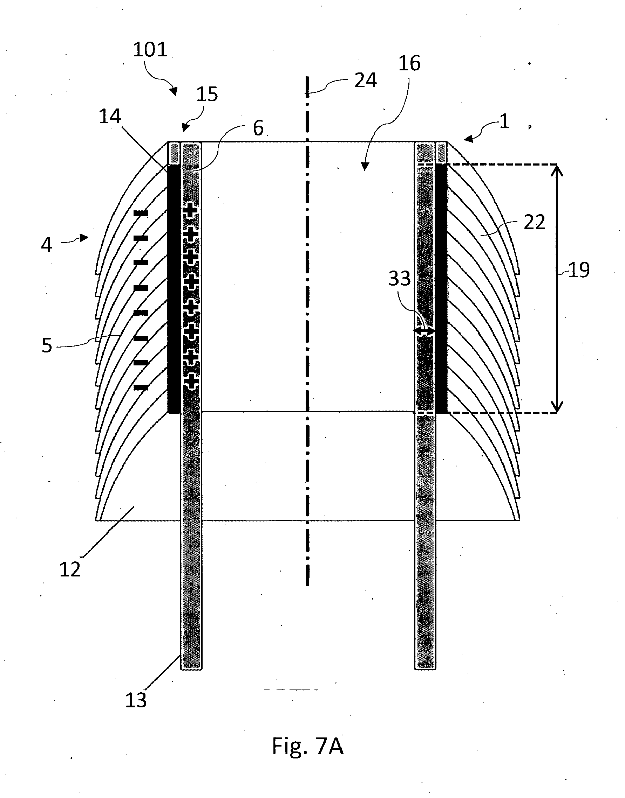

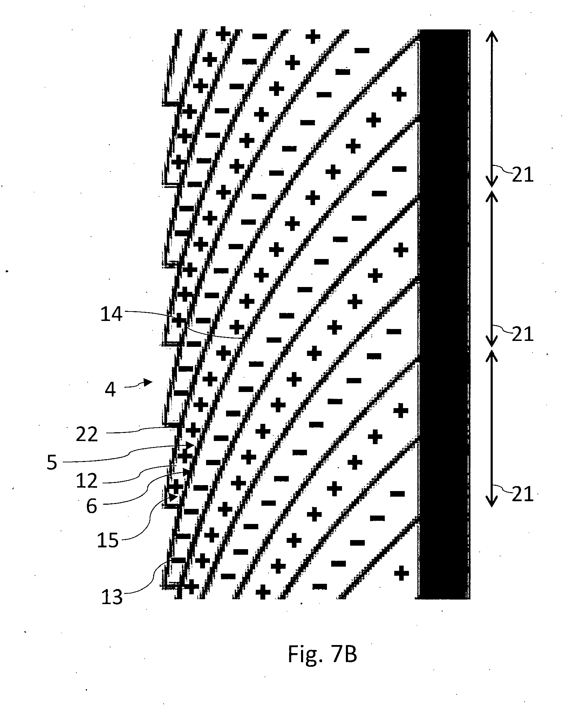

The elongated support 1 is covered by partially overlapping scale elements 22 which are arranged to cover the elongated support 1 and define an outer layer 38. Concentrically nested within the outer layer 38 there is a flexible layer 23 which encloses a trigger element 2. A cross-section of the apparatus 101 in Fig. 2C shows the outer layer 38 arranged radially outside. The outer layer 38 is formed by multiple scale elements 22 having surfaces arranged radially to each other on the elongated support 1. The scale elements22 partially overlap with circumferentially neighbouring scale elements 22 and longitudinally neighbouring scale elements 22. The scale elements 22 of the outer layer 38 form at least one first conducting layer which is separated by a thin dielectric layer from at least one second conducting layer as in Fig. 7A. The neighbouring scale elements 2,2 of the outer layer 38 can also be configured to have a voltage difference applied to individual scale elements 22 as in Fig. 7B. In this embodiment however, the scale elements 22 are conductively connected to each other to form the first conducting layer.

The cross-sectional view of the apparatus 101 in Fig. 2C shows four navigation elements 3 realised by tendons which allow deflecting the apparatus 101 or a section of the apparatus 101 in four different lateral directions and any combination of these directions.

In this embodiment the apparatus 101 comprises a flexible layer 23 in which the navigation elements 3 are embedded. The flexible layer 23 forms at least one conductive path between the trigger element 2 and the at least one second conducting layer 38. This embodiment of the apparatus 101 has a plurality of second conductive layers or elements at least partially non-conductively separated from each other along the longitudinal direction of the device, such that a voltage can be applied to these second conductive layers or elements by the insertion of the trigger element 2 to longitudinal sections of the apparatus 101. Thus, a friction is only created in the areas where the trigger element 2 has been arranged to apply said voltage difference.

The at least one conductive path hence can be used to apply an electrical voltage and adjust the stiffening of the elongated support 1 by increasing the friction between the conductive layers. The flexible layer 23 may be formed of conducting material and define the conductive path itself. Alternatively the flexible layer 23 may be formed by an insulating material comprising a conducting material between the trigger element 2 and the second conducting layer 38, so that at least one electrically conductive path can be formed. The conducting material can assume the form of a radial, in particular cylindrical, metallic insert positioned on the trigger element 2 forming a conductive path to second conducting layer 38.

The flexible layer 23 also houses the navigation elements 3 and adapts the flexural stiffness of the apparatus 101 to the field of application.

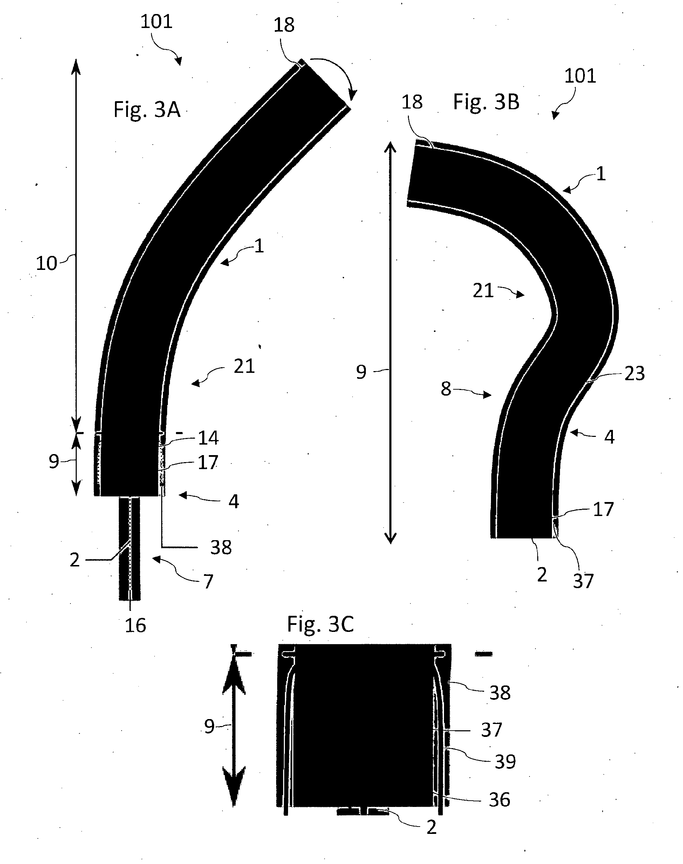

Figures 3A and 3B show a longitudinal cross-section of an alternative embodiment of an apparatus 101. Figure 3A shows a part 9 of the elongated support 1 in a locked configuration and a separate part 10 of the elongated support 1 in a flexible configuration. A bendable section 21 is configured to be reversibly convertible from the flexible configuration to the locked configuration along a freely selectable partial length of the bendable section 21.

A locking arrangement 4 of the apparatus 101 can be activated/deactivated by advancing/retracting a trigger element 2 along a longitudinal direction of the apparatus 101. However, the locking arrangement 4 can extend along only a certain length or certain parts of the apparatus 101.

In this embodiment the bendable section 21 can be converted from the flexible configuration to the locked configuration by activating the locking arrangement 4 in the form of a first and multiple second conducting layers separated by at least one insulating layer (see Fig. 7A and Fig. 7B).

The locking arrangement 4 can be activated by advancing the trigger element 2 which is nested within the outer layer 38 of the elongated support 1. The trigger element 2 hence can be used to increase and decrease the friction between the first and second conducting layer.

The locked configuration can in turn be reversely converted to the flexible configuration by retracting the trigger element 2 relative to the outer layer 38.

In this embodiment of the apparatus 101 the trigger element 2 can be advanced from a proximal end region 17 to a distal end region 18 by sliding along the longitudinal axis of the apparatus 101.

Figure 3C shows that the trigger element 2 is evertable such that an evertable sheath 36 is everted outwardly to be placed adjacent to the outer layer 38 when the trigger element 2 is advanced. An evertable trigger element 2 can preferably be advanced without translational movement of the outer surface part of the trigger element 2 being required. Thus, the frictional force when moving the trigger element 2 forwards would be minimized. The circular outwardly directed dashed arrows in Fig. 3C indicate the forward propagation movement of such an evertable trigger element 2. The backwards propagation movement of the evertable trigger element can be carried out by inverting it inwardly again. When the trigger element 2 is moved axially, the evertable sheath 36 at the distal end is everted outwardly and is lined against an inner surface of the outer layer 38.

The locking arrangement 4 functions by applying a voltage difference to conducting layers to increase friction (see Fig. 7A and Fig. 7B). The trigger element 2 is made of or comprises a conducting layer or conducting element such that the trigger element 2 can apply a voltage difference to at least one second conducting layer or define at least one second conducting layer. By everting the trigger element 2 the outwardly facing surface 39 is directed towards a first conducting layer of the outer layer 38 with a dielectric layer arranged therebetween. Thereby, the part 9 of the apparatus 101 with an introduced locking element 2 can be stiffened when a voltage difference is applied to the first and second conducting layer.

The trigger element 2 can be everted from the proximal end region 17 of the apparatus 101 such that an inner cavity 37 sealed off by the surrounding evertable sheath 36 of the trigger element 2 by pressurizing the sheath 36.

The trigger element 2 can optionally be adjusted to frictionally engage the outer layer 38 when fully everted or inhibited from further everting at an intermediate everted state by adjusting the inflation pressure within the inner cavity 37.

The trigger element 2 comprises a lumen 16 for introducing surgical instruments or implants at the proximal end region 17 to a target site beyond the distal end region 18 (not shown in the figures).

The first position 7 in Fig. 3A denotes a retracted state of the trigger element 2. The second position 8 in Fig. 3B denotes an advanced state of the trigger element 2 which was moved relative to the outer layer 38. The part 10 of the bendable section 21 in the flexible configuration in Fig. 3A is bendable. The part 9 of the bendable section 21 is stiff and not bendable due to the increased friction between the conducting layers.

The part 9 of the bendable section which is in the locked configuration in Fig. 3B extends over the entire length of the bendable section 21 due to a completely advanced trigger element 2.

The apparatus 101 can preferably take on complex geometric forms that form different bending angles along parts of the apparatus 101 of different lengths. The conducting layers are subject to a different voltage and are separated by at least one insulating layer. The conducting layers function similar as an electroadhesive clutch. Such an electroadhesive clutch can function on low power since there is no substantial current across the insulating layer between the conducting layers.

The embodiments according to Fig. 2A and Fig. 2B and the embodiments of Fig. 3A - Fig. 3C can be combined. For this purpose, the navigation elements 3 according to Fig. 2A and Fig. 2B can be divided into subsections of the cross-section of the apparatus 101. Individual cross-sectional subsections can be configured to house the navigation elements 3, in particular tendons, and define a cavity for insertion of a locking element 2 which is optionally evertable.

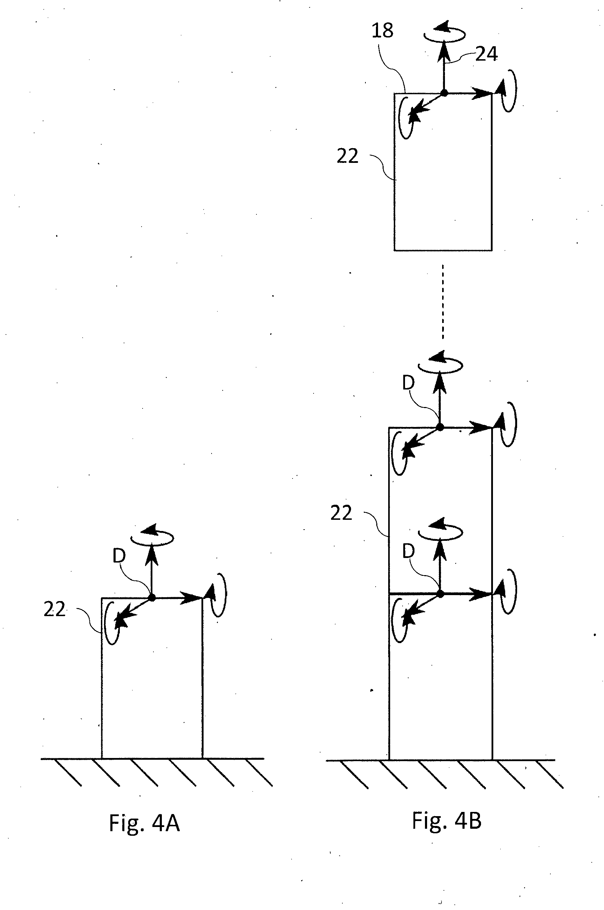

Figures 4A and 4B show a schematic illustration of discrete flexible segments representing the scale elements 22. The smallest discrete step for adjusting the flexible section of the apparatus 101 is achieved by adjusting a single scale element 22 or a single row of scale elements along the circumference. The shape generating feature due to incremental alignment of the discrete flexible segments in form of scale elements 22 is illustrated by alignment in 3 rotational degrees of freedom D and in 3 translational degrees of freedom D of successive scale elements 22.

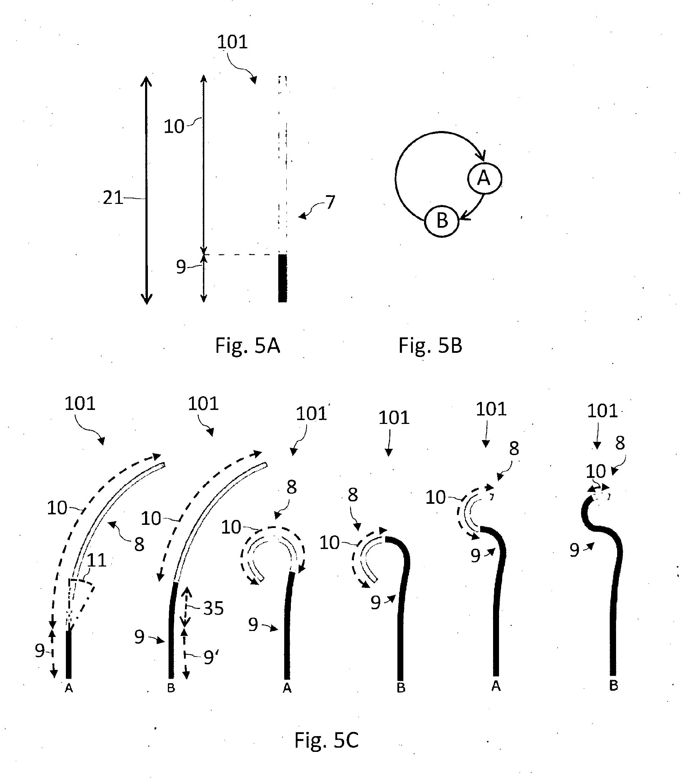

Figures 5A to 5B illustrate an iterative process of converting the apparatus 101 from a first position 7 assuming a straight configuration to a desired geometric shape.

A part 9 of the apparatus 101 which in a locked configuration is increased stepwise by advancing a trigger element (see e.g. Fig. 1) along a part 10 of a bendable section 21 which still is in a flexible configuration. The process steps are schematically illustrated in Fig. 5B. In step A a uniform bending angle is set for the part 10 of the bendable section 21 which is in the flexible configuration by operating navigation elements. The navigation elements can only deflect the part 10 of the bendable section 21 which is in the flexible configuration. In step B the trigger element is advanced along the apparatus 101 to convert a part of the previously bent section 21 from the flexible configuration to the locked configuration. Thereby, the part 9 of the bendable section 21 which is in the locked configuration is increased in a distal direction sequentially. The part 10 of the bendable section 21 in the flexible configuration is sequentially converted to the locked configuration after being deflected to the desired curvature in each iterative step. The arrow connecting step B to A illustrates that these steps are repeated until the apparatus 101 assumes a desired geometric shape.

This mechanism is schematically illustrated in Fig. 5C which shows the various configurations assumed by the bendable section in the iterated steps A and B. A part 10 of the bendable section 21 which is in the flexible configuration can be bent at angles 11. A part which is in the flexible configuration is then converted to a locked configuration over a selectable length 35 in step B by advancing the trigger element inside the apparatus 101. The length 35 is selectable by a user by advancing the trigger element to a desired location. Thereby the former part 9' of the bendable section which is in the locked configuration is extended in the distal direction by the selected length 35 to such as to form an extended part 9 which is in the locked configuration. The angles 11 can be adjusted by a user in each iterative step so that the desired curvature for the next part 9' to be locked can be adjusted.

In this embodiment the second positions 8 are defined by different longitudinal advancement positions of the trigger element with a first section on the trigger element relative to a second section on the elongated support.

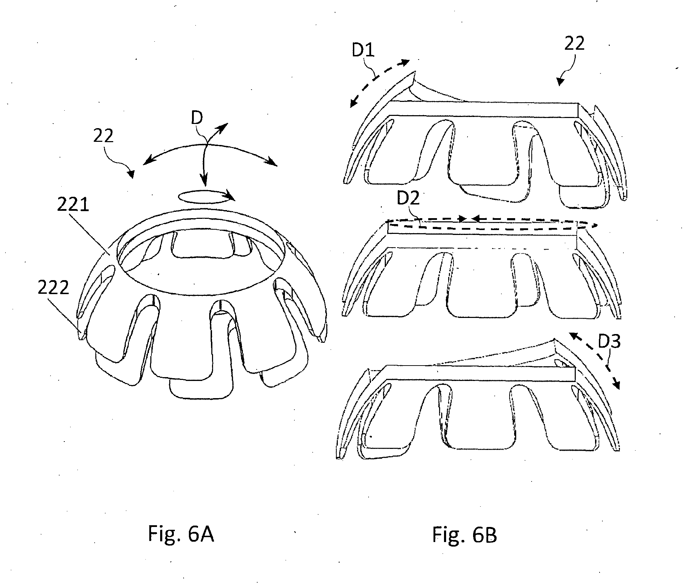

Figures 6A and 6B show an embodiment of scale elements 22 which are tiltable and rotatable. The range of the tilting angle of the scale elements is limited by neighbouring scale elements 22 which is exemplary shown in Fig. 6A by the relative position of a first partly spherical scale element 221 in relation to a second partly spherical scale element 222.

In Fig. 6 the scale elements 22 are decreasing in thickness radially outwardly from an axis of the apparatus and assume a uniformly bent shape at an angle from the axis of the apparatus. Due to such shape of the scale elements 22 neighbouring scale elements 22 can move with respect to each other and hence contact each other independent of the bending angle of the bendable section.

At a predefined maximum bending angle, the scale elements 22 may block each other to prevent further deflection of the apparatus. Preferably, this maximum bending angle is defined such as to retain a minimal remaining contact surface area between two neighbouring scale elements.

The scale element 221 and 222 are freely movable relative to each other similar to ball joints, such that they can be adjusted by an operator by a navigation element or by deflecting the apparatus by advancing it against an obstacle.

The scale elements 22 are radially stiff. Thereby, an elongated support can be deflected without being compressible in a radial direction.

The ball joints are created by radially overlapping sections of the scale elements 22. The sections comprise a ball joint head with an annular shape which is partially enclosed by a counterpart forming a socked for the head. In this embodiment the scale elements 22 extend annularly and can be arranged concentrically to the apparatus. However, the scale elements 22 could also be attached to an outer surface of the elongated support in partial segments in circumferential direction of the apparatus.

Figure 7A shows an embodiment of a locking arrangement 4 of an elongated support 1 with scale elements 22 according to Fig. 6A and Fig. 6B. The locking arrangement 4 extends over a partial length 19 of the apparatus 101, and may also extend along the complete apparatus 101, or multiple partial ranges of the apparatus 101.

In this embodiment the scale elements 22 together form a first conducting layer 12 which is separated by a thin dielectric layer 14 from a second conducting layer 13. The first and second section 5, 6 in Fig. 7A are defined by the radially spaced apart first and second conducting layers 12, 13 which are movable relative to each other by bending the apparatus. The second section 6 defining, the second conducting layer 13 has a radial width 33 and coaxially encloses a lumen 16 along the longitudinal axis 24 of the apparatus 101. The scale elements 22 contact each other irrespective of the assumed bending angle of the apparatus 101. The first and second conducting layer 12, 13 are movable relative to each other in a longitudinal direction of the apparatus relative to each other. The length 19 or parts thereof is reversibly convertible from the flexible configuration to the locked configuration..The flexible configuration can be converted to the locked configuration by a trigger element applying a voltage difference on the conducting layers 12, 13. A negative voltage can be applied to the first conducting layer 12 and the second conducting layer 13 can be applied with a positive voltage.Thereby, an attractive electrostatic force is generated between the conducting layers 12, 13 compressing them together. This electrically controllable compressional force creates an indirect friction between the conducting layers 12, 13, via the dielectric layer 14. The movement of the conducting layer 12, 13 relative to each other can be impeded or prevented depending on the voltage difference.

Alternatively, one of the conducting layer 13 can also be arranged on a moveable trigger element (not shown in the figure) which can be positioned with respect to the conducting layer 12 to only partially convert the flexible configuration 10 to a locked configuration as shown in Fig. 3A, Fig. 3B, and Fig. 5A to Fig. 5C.

A frictional interface 15 is formed between the conducting layers 12, 13 by the insulating dielectric layer 14. In this embodiment the first conducting layer 12 is arranged to form a mantle surface of the apparatus 101 such that the conducting layers 12, 13 can extend over a maximum surface. The friction of conducting layers 12, 13 can be adjusted from a continuous range proportionally to the strength of the applied electric field.

Figure 7B shows an alternative embodiment of multiple bendable sections 21 each comprising two scale elements 22. The bendable sections 21 are arranged in the longitudinal direction neighbouring each other.

The scale elements 22 are placed adjacent to each other, contacting each other along a spherical surface. The scale elements 22 comprise a conducting layer covered by a dielectric layer 14 such that they are not conductively connected to each other.

Each scale element 22 defines an upper first and a lower second conducting layer 12, 13. In Fig. 7B neighbouring scale elements 22 are configured to be applied with a relative voltage difference. The voltage can be applied through individual conducting paths conductively connecting each scale element 22, e.g. by a flexible printed circuit board. Thereby, multiple frictional interfaces 15 are formed between multiple first conducting layers 12 having a negative voltage and multiple second conducting layers 13 having a positive voltage. A first section 5 is defined by a surface of the first conducting layer 12 facing the dielectric layer 14 and a second section 6 is defined by an oppositely arranged surface of the second conducting layer 13 facing the dielectric layer 14. The resulting frictional interface 15 has a first conducting layer 12 with a first section 5 adjacent the dielectric layer 14 which is contacted from the other side by the second section 6 of the second conducting layer 13. The scale elements 22 defining the conducting layers 12, 13 can be movable by tilting relative to each other such that the first section 5 and the second section 6 move relative to each other when the curvature of the bendable section 21 is changed.

Although illustrated as a repeating series of a single positively charged conducting layer 12 and a single negatively charged layer 13, it is readily understood that any given sequence of differently charged conducting layers 12, 13 could be generated using the same embodiment.

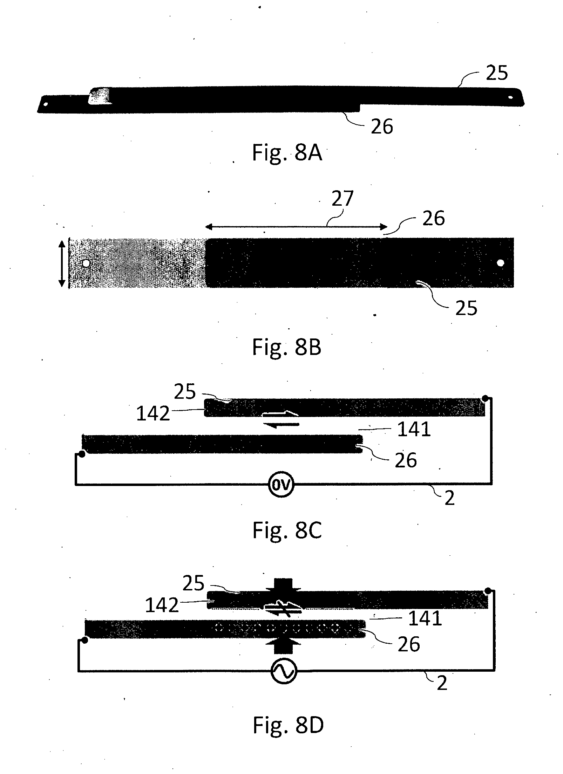

Figures 8A to 8D schematically show the mechanism which can be used in some embodiments of the invention to increase friction of adjacent conducting layers by electromagnetic braking. The mechanism is illustrated by moving a first conducting strip relative to a second conducting strip. The strips are separated by at least one dielectric layer, preferably a first dielectric layer 141 on the first conductive strip 25 and a second dielectric layer 142 on the second conductive strip 26, as shown by Fig. 8A to Fig. 8C. Figure 8B shows a frictional interface formed by an overlapping area 27 of the two conductive strips 25, 26. The conductive strips 25, 26 can move at a low friction relative to each other as illustrated by the arrows pointing to both sides in fig. 8C if no voltage is applied by a trigger element 2. Once a voltage is applied by the trigger element 2 in Fig. 8D, the normal forces and thus the friction between the strips 25, 26 is increased, as illustrated by the crossed out arrows. The voltage can be applied selectively in a time interval such that the friction can be switched on and off by the trigger element 2 or adjusted continuously.

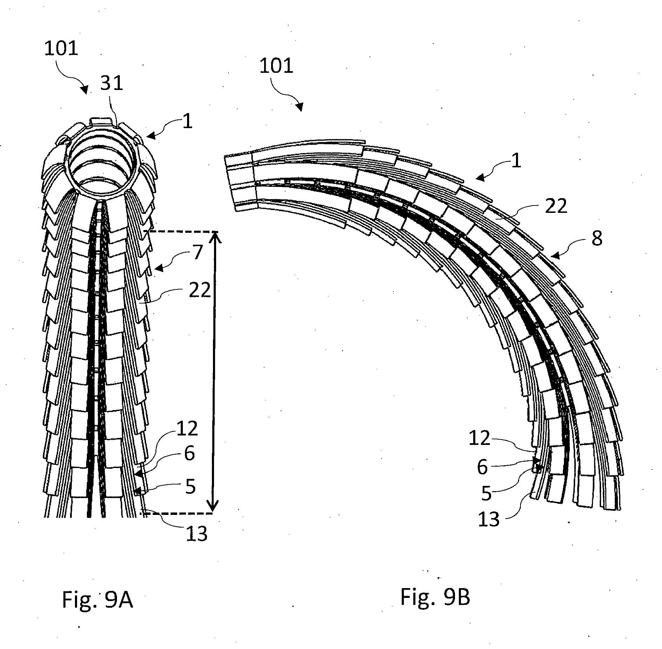

Figures 9A and 9B show an embodiment of the apparatus 101 according to Fig. 7B with scale elements 22 in a first position 7 and second position 8. The apparatus 101 in the first position 7 has a straight shape. In this embodiment the first section 5 and second section 6 are formed by the surfaces of scale elements 22 arranged at a neighbouring longitudinal positions along the longitudinal direction of the apparatus 101 contacting the dielectric layer (see Fig. 7B). Figure 9B shows that the first section 5 and second section 6 are moved relative to each other if the apparatus 101 assumes a second position 8 with a bent shape.

The scale elements 22 limit the maximum bending angle by form fittingly interlocking or blocking each other at a certain curvature of the apparatus 101.

In this embodiment the scale elements 22 are arranged in circumferential rows such that the scale elements 22 are directed partially radially outwardly from the apparatus 101. Scale elements 22 are also arranged at an angle below 45° with respect to a longitudinal axis of the apparatus 101. Each row of scale elements 22 comprises nine scale elements 22 which are connected by a connecting ring 31. The scale elements 22 are elongated to partially overlap one or more other circumferential row of other scale elements 22 and are electrically separated by being coated with an insulating layer. The scale elements 22 comprise stainless steel. The scale elements 22 have essentially the same radial size along their longitudinal axis. The embodiment in Fig. 9A and Fig. 9B only shows a part of the apparatus 101 which is covered by scale elements 22. In this embodiment of the apparatus 101 the elongated support 1 can be converted over the entire length from the flexible configuration to the locked configuration. When the apparatus 101 or a longitudinal subsection of the apparatus 101 is in the locked configuration further deflection is inhibited by the increased friction.

Alternatively, the scale elements 22 can be connected directly to other scale elements 22 by contacting each other to form a conducting layer (see Fig. 7A).

Furthermore, the scale elements 22 are not necessarily individually fixedly connected to the remainder of the apparatus 101 but may be simply arranged coaxially to it in a sleeve like manner.

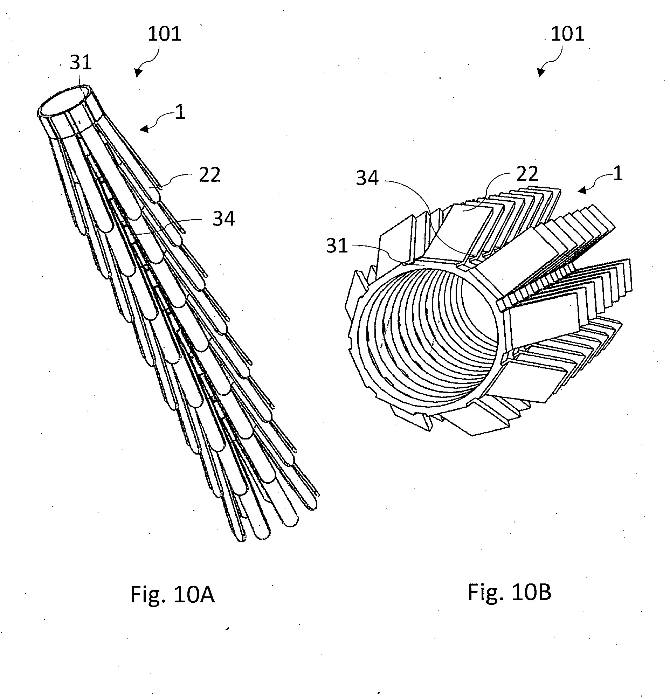

Figure 10A and 10B show an alternative embodiment of the apparatus 101 with scale elements 22 in a perspective side view and top view. The scale elements 22 are arranged at a smaller angle to a longitudinal axis than in Fig. 9A to Fig. 9C. The scale elements 22 have rounded end areas such that the risk of unintentional interlocking is minimized. The perspective top view in Fig. 10B shows that the scale elements 22 are arranged at an angle to the longitudinal axis of the apparatus 101. The individual scale elements 22 in this embodiment are arranged in rows one above the other along the longitudinal axis of the apparatus 101 such that an axial gap 34 between the scale elements in a circumferential direction extends along the longitudinal direction of the apparatus 101. The elongated support 1 can comprise a tubular member which connects the scale elements 22.

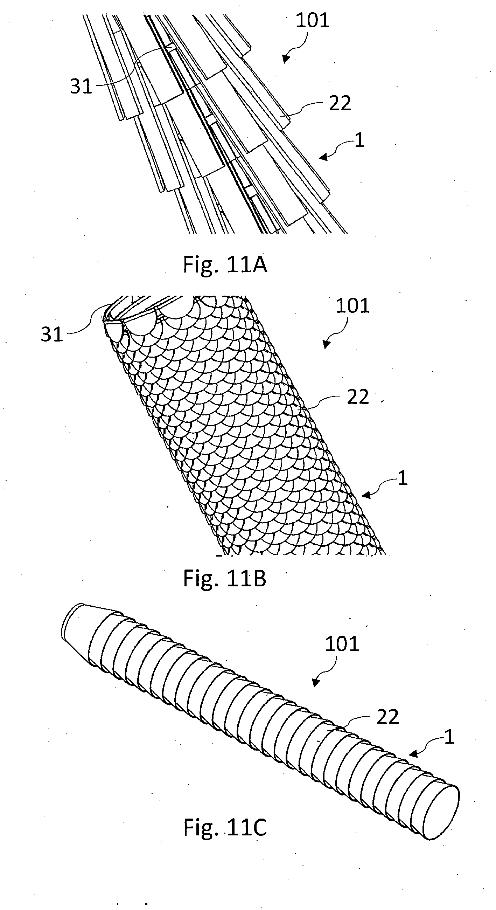

Figures 11A to 11C show further alternative embodiments of the apparatus 101 with different scale elements 22. In Fig. 11B the scale elements 22 have a shape of a semicircle and are arranged on the outside of the elongated support 1 similarly to fish scales. Connecting rings 31 are arranged circumferentially along a certain longitudinal section of the apparatus 101. The connecting rings 31 carry multiple scale elements 22 so that the individual scale elements 22 on the connecting rings 31 can be distanced by a small gap. The semi-circular scale elements 22 are arranged in an alternating orientation such that a neighbouring row of scale elements is circumferentially offset to the neighbouring scale elements 22 by half of the width of the scale elements 22.

The scale elements 22 in Fig. 11C have a conical shape concentric to the longitudinal axis of the apparatus 101. The scale elements 22 are stacked on top of each other along the longitudinal axis. The scale elements 22 can also vary in thickness (not shown in Fig. 11A to Fig. 11C) and the scale elements can in particular decrease in thickness laterally outwardly from the apparatus 101 and assume a bent shape, in particularly a laterally inward bent shape (see Fig. 7).

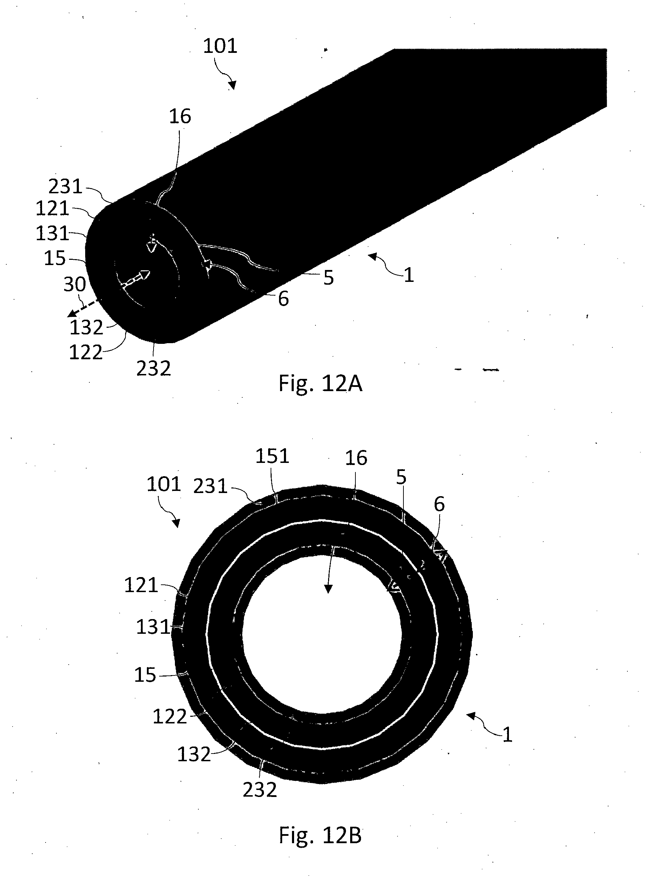

Figures 12A and 12B show an alternative embodiment of the apparatus 101. An outermost layer 231 and an innermost layer 232 are formed as protective flexible layers enclosing two conducting layers 121, 122. Between the conducting layers 121, 122, two dielectric layers 131, 132 are arranged. All layers are arranged coaxially to each other. The elongated support 1 defined by these layers coaxially encloses a lumen 16 which can be used as a working channel for introducing deployable structures or surgical instruments into the patient's body. The two conducting layers 121, 122 are separated by the dielectric layers 131, 132 which consist of an electrically insulating piezo film. The adjacent dielectric layers 131, 132 form a frictional interface 15 at their contacting surface. Applying a voltage between the two conducting layers 121, 122 leads to an increase in friction between the dielectric layers 131, 132 at the frictional interface 15. Thereby, the friction between the conducting layers 121, 122 is indirectly increased. A first section 5 and a second section 6 have a radial width illustrated by the two-headed dashed arrows in Fig. 12B. As schematically illustrated by the dashed arrow in in Fig. 12A the first section 5 is translationally movable 30 relative to the second section 6 in the longitudinal direction.

The inner and outer flexible protective layers 231, 232 of the elongated support 1 can comprise a flexible insulating material. This embodiment of the apparatus 101 does not comprise any scale elements. The voltage difference between the conducting layers 121, 122 can preferably be supplied by a voltage supply, in particular an external control element (not shown in the figures). If voltage differences are applied to the conducting layer 121, 122, the first section 5 and second section 6 are translationally not movable to each other due to an increased friction. In such a locked configuration of the elongated support 1, the elongated support 1 has a high resistance to bending. If the voltage difference is removed, the first section 5 and second section 6 are translationally movable in longitudinal direction relative to each other again and the apparatus 101 becomes bendable again.

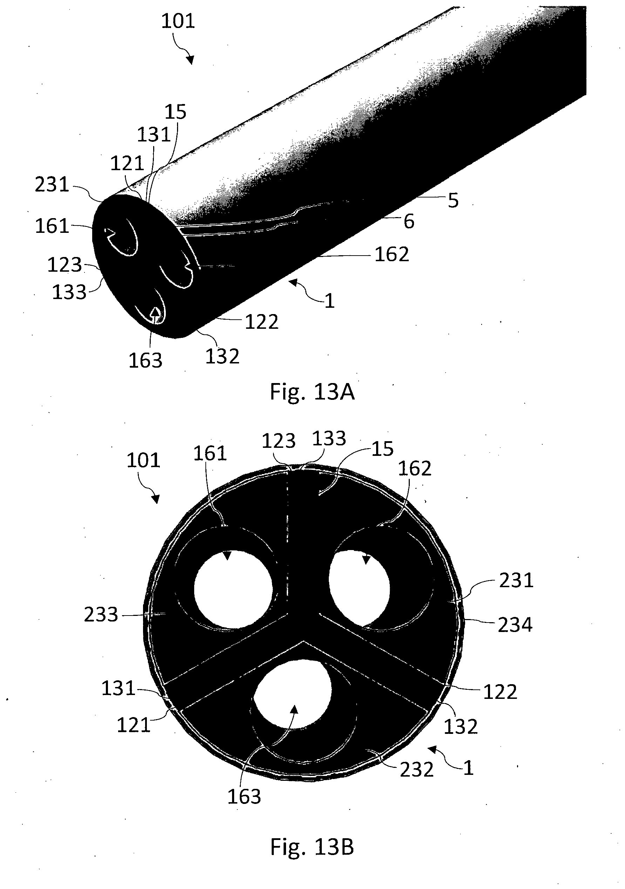

Figures 13A and 13B show an alternative embodiment of the apparatus 101 with conducting layers 121, 122, 123 which are arranged in planes from the longitudinal axis of the apparatus 101. Thereby, the conducting layers 121, 122, 123 divide the cross-section of the apparatus (see Fig. 13A) into three equally sized sections. Each section is enclosed by a flexible protective layer 234 and a dielectric layer 131, 132, 133. Each of these sections comprises a lumen 161, 162, 163 which can be used as a surgical working channel. The dielectric layers 131, 132, 133 are arranged adjacent to each other and form frictional interfaces 15. The friction between the conducting layers 121, 122, 123 can be indirectly increased, by increasing the friction between the dielectric layers 131, 132, 133 when a voltage difference is applied to the conducting layers 121, 122, 123 separated by dielectric layers 131, 132, 133. If the voltage is removed, the dielectric layers 131, 132, 133 are not prevented or inhibited from moving relative to each other in a longitudinal direction of the elongated support 1 anymore. The lumina 161, 162, 163 are centrally positioned within a flexible support 231, 232, 233. Surgical or endoscopic elements can be inserted through the lumina 161, 162, 163. The protective layer 234 is non-conductive so that the conducting layers 121, 122, 123 are isolated from the surroundings. The conducting layers 121, 122, 123 in Fig. 13A and Fig. 13B are integrally formed. The' conducting layers 121, 122, 123 form continuous layers along the longitudinal direction of the apparatus 101 and are arranged parallel to each other. The three sections can be movable in the longitudinal direction 30 relative to the rest of the apparatus 101. One of the three sections can define a first section 5 and the rest of the apparatus 101 can define a second section 6.

## Claims

1. Apparatus (101) for insertion into a patient's body, preferably a body duct, in minimally invasive procedure, the apparatus comprising an elongated support (1) and a trigger element (2), wherein
the elongated support comprises at least one first section (5) and at least one second section (6) which are moveable with respect to each other between a first position (7) and a second position (8),
the elongated support (1) has at least one locking arrangement (4) configured to reversibly
(i) increase friction between the at least one pair of first section (5) and second section (6) in a locked configuration, thereby inhibiting a relative movement between the first section (5) and second section (6) due to the increased friction and locking the elongated support (1) in at least one of the first position (7) and second position (8) and
(ii) to decrease friction between the first section (5) and second section (6) in a flexible configuration, thereby allowing a relative movement between the first section (5) and second section (6)
and the locking arrangement (4) is activatable by the trigger element (2).

2. Apparatus (101) according to claim 1, wherein the trigger element (2) is an elongated element and one of the trigger element (2) and the elongated support (1) is nested within the other of the trigger element (2) and the elongated support (1),
the trigger element (2) and the elongated support (1) being slidable relative with respect to each other.

3. Apparatus (101) according to one of the claims 1 or 2, wherein a bendable section (21) encompassing the first section (5) and the second.section (6) is bendable along a preferably uniform bending angle (11) and wherein the first position (7) defines a straight arrangement of the bendable section (21) and the second position (8) defines a bent arrangement of the bendable section (21).

4. Apparatus (101) according to claim 3,
wherein the trigger element (2) is configured to at least partially activate the locking arrangement (4) if advanced in a longitudinal direction of the elongated support (1) to an area neighbouring the bendable section(21) and
the trigger element (2) is configured such that displacing, in particular retracting, the trigger element (2) in longitudinal direction from the bendable section (21) of the elongated support (1) at least partially deactivates the locking arrangement (4).

5. Apparatus according to one of the claims 3 to 4, wherein the apparatus (101) comprises at least one navigation element (3), in particular two pull-wires, in particular preferably at least three pull-wires, for applying bending forces in radial directions of the apparatus (101) for deflecting and/or steering the apparatus(101) and bending the bendable section (21), when the first section (5) and second section (6) are in the flexible configuration.

6. Apparatus according to one of the preceding claims, wherein the locking arrangement (4) has a first conducting layer (12) and a second conducting layer (13), wherein the friction between the first conducting layer (12) and the second conducting layer (13) is adjustable by applying a difference of electric potential between the first conducting layer (12) and second conducting layer (13).

7. Apparatus according to one of the preceding claims, wherein the first.conducting layer (12) or the second conducting layer (13) is positioned on or adjacent the trigger element (2) and the trigger element (2) is adapted to generate a difference of electric potential between the conducting layers (12, 13).

8. Apparatus according to claim 7, wherein the locking arrangement (4) comprises multiple first conducting layers (12), preferably dielectric layers (14) and/or second conducting layers (13) configured so that at least two frictional interfaces (15) are formed.

9. Apparatus according to one of the preceding claims, wherein the apparatus (101) comprises at least one elongated lumen (16) which preferably extends from a proximal end region (17) of the apparatus (101) to a distal end region (18) of the elongated support (1) and wherein the trigger element (2) is preferably axially moveable within the lumen (16).

10. Apparatus according to one of the preceding claims, wherein the locking arrangement (4) is disposed (i) along the entire length of the apparatus (101), (ii) only partially along the length of the apparatus (101), or (iii) along a plurality of partial lengths (19) of the apparatus (101).

11. Apparatus according to one of the preceding claims, wherein the maximum bending angle (11) is 14°, in particular 35°, in particular preferably 45°, along a longitudinal length of the apparatus of 1 cm..

12. Apparatus according to one of the preceding claims, wherein the locking arrangement (4) has multiple scale elements (22) which are at least partially overlapping along the longitudinal and/or the circumferential direction of the apparatus (101).

13. Apparatus according to claim 12, wherein the scale elements (22) are configured to be at least partially tiltable in two degrees of freedom away from a longitudinal axis of the apparatus (101) and preferably rotatable in circumferential direction of the apparatus (101).

14. Apparatus according to claims 12 or 13, wherein the scale elements (22) are configured to increase the friction between the first section (5) and the second section (6), the first section (5) and the second section (6) are separate from the scale elements (22)
or the scale elements (22) include the first section (5) and the second section (6).

15. Apparatus according to claims 13 to 15, wherein the scale elements (22) are contacting each other in the first position (7) and in the second position (8), and optionally consist of or comprise a conductive material.

16. Apparatus according to any of the preceding claims, wherein the elongated support (1) has at least one of the following features: (i) the elongated support (1) has a closed surface along the longitudinal direction, (ii) the elongated support (1) is radially stiff, (iii) the elongated support (1) is configured to have a uniform flexural rigidity and (iv) the elongated support (1) has essentially the same cross-section along its longitudinal axis.

17. Apparatus according to any one of the preceding claims, wherein the trigger element (2) is configured to actuate a volume modification element which is expandable and/or collapsible in a radial direction of the apparatus (101) by inflation pressure or due to a piezoelectric effect.

18. Apparatus according to any of the claims 5 to 17, wherein the apparatus comprises an external control element which is configured to be situated outside of the patient in the proximal end region (17) of the apparatus (101), the control element (12) being configured to (i) operate the navigation element (3), or (ii) activate and/or deactivate the trigger element (2), (iii) optionally to advance and/or retract the elongated support (1) or trigger element (2) relative to the elongated support (1) or trigger element (2).
